# EUROPÄISCHE PATENTSCHRIFT

(11) **EP 1 077 072 B1**
(45) Veröffentlichungstag und Bekanntmachung des Hinweises auf die Patenterteilung: **12.11.2003**
(21) Anmeldenummer: 00108986.1
(22) Anmeldetag: 27.04.2000
(51) Int. Cl.: A61L 27/38, C12N 5/08

(54) **In vitro-Verfahren zum Herstellen einer homologen Herzklappen- oder Gefässprothese**
In vitro process for the preparation of heart valve or vessel prothese
Procédé de préparation in vitro de prothèse de valve cardiaque ou de vaisseaux

(30) Priorität: 29.04.1999 DE 19919625
(43) Veröffentlichungstag der Anmeldung: 21.02.2001
(73) Patentinhaber: Symetis AG, 8005 Zürich (CH)
(72) Erfinder: Symetis AG, 8005 Zürich (CH)
(74) Vertreter: Grünecker, Kinkeldey, Stockmair & Schwanhäusser Anwaltssozietät

(56) Entgegenhaltungen:
- WO-A-94/25584
- WO-A-97/49799
- DE-A- 19 828 726
- US-A- 5 855 610
- SHINOKA T. ET AL: "Tissue engineering heart valves: valve leaflet replacement study in a lamb model" THE ANNALS OF THORACIC SURGERY, Bd. 60, Nr. 6, 1995, Seiten s513-s516, XP000993044
- HOERSTRUP S.P. ET AL: "New pulsatile bioreactor for in vitro formation of tissue engineered heart valves" TISSUE INGENEERING, Bd. 6, Nr. 1, Februar 2000 (2000-02), Seiten 75-79, XP000993059
- ZUND G. ET AL: "The in vitro construction of a tissue engineered bioprosthetic heart valve" EUROPEAN JOURNAL OF CARDIO-THORACIC SURGERY, Bd. 11, 1997, Seiten 493-497, XP000993050
- HOERSTRUP S.P. ET AL: "Fluorescence activated cell sorting: a reliable method in tissue engineering of a bioprosthetic heart valve" THE ANNALS OF THORACIC SURGERY, Bd. 66, Nr. 5, 1998, Seiten 1653-1657, XP000993051
- Sodian, R., et al.: TISSUE ENGINEERING, Vol. 5, No. 5, 1999, pp. 489-493
- Sodian R. et al.: Abstract Book, 2nd Bi-Annual Meeting of the Tissue Engineering Society 1998, p. 479, Abstract No. O-57, 1998.

## Beschreibung

Die vorliegende Erfindung betrifft ein in vitro-Verfahren zum Herstellen einer homologen Herzklappen- oder Gefäßprothese sowie eine mit diesem Verfahren herstellbare homologe Herzklappe sowie ein mit diesem Verfahren herstellbares Gefäß.

Jedes Jahr versterben alleine in den USA ca. 20.000 Patienten an den Folgen einer Herzklappendysfunktion, und mehr als 60.000 Patienten sind wegen einer bereits erkannten Dysfunktion gezwungen, ein oder mehrere Herzklappen operativ ersetzen zu lassen. Als Ersatz für die eigene Herzklappe kommen entweder mechanische oder biologische Klappenprothesen (Xenografts) in Frage, seltener werden kryopreservierte oder glutaraldehydfixierte Homografts verwendet.

Mechanische Klappenprothesen führen jedoch oft zu Fremdkörperreaktionen mit thromboembolischen Komplikationen, die durch die mit der künstlichen Herzklappe veränderten Strömungsverhältnisse im Herzen begünstigt werden. Daher ist eine lebenslange Antikoagulation des betroffenen Patienten erforderlich, die zu einer permanent erhöhten Blutungsgefahr führt. Eine weitere, oft lebensbedrohliche Komplikation bei Patienten mit einer mechanischen Herzklappe sind Infektionen.

Bei den Xenografts handelt es sich meistens um Schweineklappen oder um Klappen aus bovinem (Rinder) Gewebe, die mittels Glutaraldehyd behandelt und fixiert werden. Diese biologischen Klappenprothesen können mit guten Ergebnissen bei älteren Patienten eingesetzt werden, neigen aber zur Degeneration nach nur ca. 12 bis 15 Jahren, so daß sie für jüngere Patienten in der Regel nicht in Frage kommen. Darüber hinaus neigen Xenografts zur Calcifizierung, weshalb sie zum Einsatz bei Kindern und jungen Menschen, die einen erhöhten Calciumstoffwechsel aufweisen, zusätzlich ungeeignet sind. Weiterhin besteht bei Xenografts im Vergleich zum gesunden Herzen eine erhöhte Infektionsgefahr. Schließlich stellen sie ebenfalls körperfremdes Gewebe dar, das mit einer gewissen Wahrscheinlichkeit vom körpereigenen Immunsystem als fremd erkannt wird und damit lebensbedrohliche Immunprozesse auslösen kann.

Als dritte Möglichkeit stehen Homografts, d. h. aus humanen Spendern isolierte, fixierte Herzklappen zur Verfügung. Homografts sind zwar gegen Infektionen relativ resistent, stellen jedoch ebenfalls körperfremdes Gewebe dar, das mit einer gewissen Wahrscheinlichkeit Immunreaktionen hervorruft. Darüber hinaus neigen Homografts ebenso wie Xenografts zur Calcifizierung und unterliegen daher einer erheblichen Degeneration, die in der Regel eine Reoperation nach 7 bis 12 Jahren erforderlich macht. Homografts stehen darüber hinaus im Rahmen des allgemeinen Organspendermangels in nur äußerst begrenztem Umfang zur Verfügung.

Neben dem bereits beschriebenen Nachteilen der bisher als Klappenersatz verwendeten Klappenprothesen, d. h. der Auslösung von Immunreaktionen, der erhöhten Infektionsgefahr, der Gefahr thromboembolischer Prozesse und der Degenerationsneigung, ist allen bisher bekannten Klappen gemeinsam, daß sie aus anorganischem Material oder fixiertem organischen Material bestehen und ihnen daher wichtige Eigenschaften lebender Matrix, z.B. die Fähigkeit zu Reparationsprozessen, zur Rekonfiguration oder zum Wachstum fehlen. Daraus folgt u.a., daß bei kindlichen Klappenpatienten bisher regelmäßig Reoperationen in Kauf genommen werden mußten. Zusätzlich zu dem jeder Herzoperation inhärenten Risiko steigt jedoch mit jeder Reoperation das Letalitätsrisiko, da durch die vorangegangenen Operationen erhebliche Verwachsungen im Thorax auftreten.

Es besteht daher ein dringender Bedarf für einen Herzklappenersatz, der die zuvor beschriebenen Nachteile vermeidet. Zu diesem Zweck ist bereits vorgeschlagen worden, künstliche Herzklappen durch "Tissue Engineering" herzustellen. Das "Tissue Engineering" befaßt sich mit der Entwicklung "biohybrider" Implantate, die im Körper zu Geweben oder gar zu ganzen Organsystemen heranwachsen. Zuerst fanden die Techniken des "Tissue Engineering" im Bereich der Hauttransplantation Anwendung. Inzwischen hat sich ihre Applikation auf andere Gewebe, wie Leber, Knorpel, Knochen und Trachea sowie intestinale und urologische Gewebe und Blutgefäße erweitert. Auch zur Herstellung biohybrider Herzklappen in Form von einzelnen Klappensegeln sind bereits beschrieben worden. Die durch "Tissue Engineering" hergestellten Herzklappensegel hatten jedoch bisher den Nachteil, daß sie inadäquate, nicht ausreichende bindegewebige Strukturen aufwiesen und daher den im Herzen herrschenden Strömungsverhältnissen nach Auflösung der biodegradablen Trägerstruktur nicht hätten standhalten können.

Wie beim Herzklappenersatz besteht auch bei der Chirurgie von Blutgefäßen ein erheblicher Bedarf an geeigneten Ersatzstrukturen. Eine ideale Lösung im Kampf gegen degenerative Erkrankungen wie die Artherosklerose steht hier ebenfalls noch nicht zur Verfügung. Der Bedarf erstreckt sich im wesentlichen auf 3 chirurgische Gebiete:
a) Herzkranzgefäße: Hier werden als Bypass-Gefäße v. a. Beinvenen und zunehmend auch arterielle Gefäße benutzt. Venen (welche physiologischerweise nur geringen Blutdrücken und Flüssen ausgesetzt sind) degenerieren unter den systemischen Druckbedingungen des Herzkranzgefäß-Systems relativ schnell, so dass nach einigen Jahre bei vielen Patienten eine Reoperation nötig wird. Arterielle Gefäße, wie z.B. die Radialarterie des Unterarms oder Arterien der inneren Brustwand (welche physiologischerweise an hohe Drücke gewöhnt sind) stehen auf Grund ihre funktionellen Bedeutung für den Organismus nur in beschränkten Maße zur Verfügung. Die Tatsache, dass heutzutage die Patienten auf Grund der verbesserten medizinischen Versorgung ein immer höheres Lebensalter erreichen und somit nach ein oder mehreren Voroperationen vermehrt zur Reoperation kommen, aggraviert den Mangel an Gefäßen zusätzlich, da in den vorangehenden Operationen die körpereigenen Venen und Arterien aufgebraucht wurden.
b) Hauptschlagader: Auch die Hauptschlagader (Aorta) unterliegt v.a. atherosklerotischer Degeneration. Zum Aortenersatz werden heute zum einen Kunststoff-Gefäßprothesen (Dacron, PTFE, etc.) verwendet. Weiterhin stehen hier, wie beim Herzklappenersatz, sogenannte "Homografts" zur Verfügung, d.h. Aortenstücke, die Organspendem entnommen werden. Die Kunststoffprothesen stellen ein Fremdmaterial dar und unterliegen einem erhöhten Risiko für Infektionen und Bildung von Blutgerinnseln. Die in dieser Hinsicht geeigneteren Homografts sind in ihrer Verfügbarkeit mangels genügender Organspender sehr limitiert.
c) Periphere Gefäße: Ebenfalls durch degenerative Prozesse wie die Atherosklerose betroffen sind die peripheren Gefäße des Körpers, wie die Halsschlagader oder Beinarterien. Hier wird neben der Möglichkeit, eine Vene als Ersatzgefäß zu nehmen (mit obengenannten Nachteilen), zunehmend Kunststoffmaterial verwendet (z.B. Dacron etc.). Dies erbringt zwar akzeptable Resultate, jedoch befindet sich bei dieser Lösung lebenslang ein Fremdmaterial im Blutkreislauf, so dass die Gefahr von Infektionen und das Risiko von Blutgerinnseln erhöht sind.

Es besteht daher ein eklatanter Mangel an geeignetem Gefäßersatz in allen erwähnten Gebieten der Gefäß-Chirurgie. Ein aus patienteneigenem Gewebe hergestellter Gefäßersatz, welcher eine lebende Struktur mit dem Potential zu Wachstum und Reparationsprozessen darstellen würde und den hämodynamischen Erfordernissen des Körperkreislaufs standhalten würde, wäre hier eine ideale Lösung.

Aufgabe der Erfindung ist es daher, Verfahren zum Herstellen von künstlichen homologen Herzklappen und Verfahren zum Herstellen von Blutgefäßen zur Verfügung zu stellen, um Herzklappenprothesen bzw. Blutgefäßeprothesen erzeugen zu können, die den in vivo herrschenden Strömungsverhältnissen gewachsen sind.

Erfindungsgemäß wird die Aufgabe durch ein in vitro-Verfahren zum Herstellen einer homologen Herzklappe oder homologer Blutgefäße zur Implantation beim menschlichen Patienten gelöst, das die folgenden Schritte umfaßt:
- Bereitstellen eines biologisch abbaubaren Trägers,
- Besiedeln des Trägers mit homologen Fibroblasten und/oder Myofibroblasten zur Ausbildung einer bindegewebigen Matrix,
- Besiedeln des Trägers/der Matrix mit Endothelzellen,
- Einbringen des Trägers/der Matrix in eine pulsatile Flusskammer, in der er/sie steigenden Flussraten und/oder Druck ausgesetzt werden kann, und
- kontinuierliches oder diskontinuierliches Erhöhen der Flussrate von einem Anfangswert von 50 - 100 ml/min bis zu einem Endwert von 2000 - 5000 ml/min und/oder des Druckes auf 120 - 240 mm/Hg.

Mit dem erfindungsgemäßen Verfahren können funktionale lebende homologe Herzklappen bzw. homologe Blutgefäße hergestellt werden, die auf Grund ihrer spezifischen bindegewebigen Struktur mit naturnahem Kollagen-, Elastin- und Glycosaminanteil zur Implantation beim menschlichen Patienten geeignet sind. Das Verfahren soll im folgenden näher erläutert werden.

In der folgenden Beschreibung bedeutet der Terminus "Träger" eine azelluläre Struktur, die, wie unten genauer erläutert wird, entweder aus synthetischen Fasern oder einem azellulären Bindegewebsgerüst besteht. Der Begriff "Matrix" bezeichnet eine bindegewebige Struktur, die neben Fibroblasten und Myofibroblasten typische Bestandteile einer Extrazellulärmatrix, nämlich Kollagen, Elastin und Glycosaminoglycane enthält. Mit Matrix bezeichnete Strukturen enthalten typischerweise keine Trägerbestandteile mehr. Die Übergangsstadien zwischen Träger und Matrix werden durch die Doppelbezeichnung "Träger/Matrix" beschrieben. "Lebend" bedeutet ein vitales Zellgewebe mit der Fähigkeit zu Wachstum, Reparation und Bildung von Extrazellulärmatrix-Proteinen.

Zur Durchführung des erfindungsgemäßen Verfahrens wird zunächst ein biologisch abbaubarer Träger bereitgestellt. Das Trägermaterial soll dabei einerseits eine gewisse Zeitlang stabil sein, um eine ausreichende Besiedlung bzw. Durchdringung mit Fibroblasten und/oder Myofibroblasten zu ermöglichen und die Ausbildung einer bindegewebigen Matrix erreichen zu können, andererseits innerhalb einer vertretbaren Zeit, die jedenfalls kleiner ist, als die Zeit, die die Bildung der homologen Klappenprothese bzw. Blutgefäßprothese in Anspruch nimmt, insgesamt oder weitgehend biologisch (hydrolytisch) abgebaut werden können. Es ist bevorzugt, daß der biologische Abbau nach ca. 8 Tagen beginnt; er sollte in der Regel nach 4 bis 6 Wochen abgeschlossen sein.

Bei dem Trägermaterial handelt es sich bevorzugt um eine aus Polymerfasern aufgebaute Struktur, um eine poröse Polymerstruktur oder ein azelluläres biologisches Gewebe. Beispiele für biologisch abbaubare Trägermaterialien sind Polyglycolsäure (PGA), Polymilchsäure (PLA), Polyhydroxyalkanoat (PHA) und Poly-4-Hydroxybutyrat (P4HB). PHA und insbesondere PH4B haben den Vorteil, dass sie sich aufgrund ihrer Thermoplastizität mittels Hitze formen und so z.B. in die Form einer Herzklappe bringen lassen. Die Polymere können sowohl rein als auch in Mischungen aus zwei oder mehreren der genannten Substanzen oder Mischungen dieser Substanzen mit weiteren biologisch abbaubaren Polymeren verwendet werden. In einer bevorzugten Ausführungsform wird ein Mischpolymer aus 85% PGA und 15% PLA verwendet. In einer weiteren bevorzugten Ausführungsform wird ein Mischpolymer aus ca. 90% PGA und ca. 10% P4HB verwendet . Dieses Mischpolymer ist bei 60-70 °C formbar und ermöglicht es daher, z.B. tubuläre Strukturen zu formen und zu verkleben oder Herzklappen vorzuformen.

Ein Beispiel für eine mögliche Herzklappenform ist in Abbildung 1 a angegeben. Herzklappen weisen in der Regel einen Außendurchmesser von 12 bis 34 mm auf und die Klappensegel sind ca. 0,2 mm bis 2,0 mm dick. Für Röhren schwanken die Innendurchmesser, je nach vorgesehener Verwendung des Implantats, zwischen 2 und 34 mm. Die Wanddicke solcher Röhren liegt bevorzugt bei 0,2 bis 2,0 mm. Ihre Länge beträgt 10 bis 200 mm. Die Abbildungen 6 und 7 zeigen eine Aufsicht auf und einen Querschnitt durch einen tubulären Träger.

Es hat sich als sinnvoll erwiesen, Träger mit einer Polymerdichte von ca. 40 bis 120 mg/cm³ zu verwenden. Unterhalb von 40 mg/cm³ ist das Polymergewebe zu labil, überhalb von 120 mg/cm³ ist das Gewebe zu dicht, um das Eindringen von Fibroblasten innerhalb eines vertretbaren Zeitraums zuzulassen. In bevorzugten Ausführungsformen beträgt die Dichte des biologisch abbaubaren Trägers 50 bis 80 mg/cm³, besonders bevorzugt 70 mg/cm³. Von den Erfindern wurde mit guten Ergebnissen ein polymerer Träger der Fa. Albany International Research, Mensville, MA, USA, mit einer Dichte von ca. 70 mg/cm³ verwendet. Die Fasern des Trägers können einen Durchmesser von 6 bis 20 um haben, bevorzugt 10 bis 18 µm. Es sind jedoch auch Gewebe mit anderen Faserstärken denkbar, die jedoch einerseits dem Träger eine gewisse Stabilität verleihen müssen, andererseits die Besiedelung und Durchdringung des Trägers mit Fibroblasten oder Myofibroblasten zulassen müssen. Weiterhin können poröse (schwammartige) Polymerformen verwendet werden. Hier haben sich Porengrößen von 80 - 240 µm als bevorzugt erwiesen. Die Poren können durch die sogenannte "Salt-Leaching" Technik erzielt werden, die dem Fachmann bekannt ist.

Statt eines synthetischen Trägers, wie zuvor beschrieben, ist die Verwendung eines azellulären Bindegewebsgerüstes denkbar. So könnte beispielsweise eine Schweineklappe oder ein Schweinegefäß chemisch azellularisiert und damit in ein immunologisch neutrales Gewebe umgewandelt werden (Bader et al., Eur. J.-Cardiothorac. Surg. 14, 279, 1998), das anschließend mit homologen Zellen besiedelt werden könnte.

Idealerweise ist der biologisch abbaubare Träger in der Form der gewünschten Herzklappe bzw. als Röhre vorgeformt. Der biologisch abbaubare Träger wird zunächst mit einer Fibroblastenpopulation und später mit einer Endothelzellpopulation inkubiert. Bei Verwendung homologer Fibroblasten und/oder Myofibroblasten und Endothelzellen, d. h. von Fibroblasten und/oder Myofibroblasten und Endothelzellen aus einem Menschen, aber nicht unbedingt dem Patienten, sollte auf gleiche HLA-Typisierung geachtet werden. Idealerweise werden diese Zellpopulationen jedoch aus dem zukünftigen Patienten gewonnen (=autologe Zellen). Die Zellpopulationen können z.B. aus peripheren Blutgefäßen, sowohl Arterien als auch Venen, gewonnen werden. Hierzu bietet sich insbesondere die Arteria radiales des Unterarmes an, die wegen der arteriellen Doppelversorgung des Armes in den meisten Fällen zur schadlosen Explantation zur Verfügung steht Alternativ können Gefäßzellen aus Blutgefäßen des Beines, z.B. der Vena saphena, gewonnen werden.Möglich ist weiter die Gewinnung von Fibroblasten oder Myofibroblasten und Endothelzellen aus homologen und/oder autologen pluripotenten Stammzellen oder genetisch manipulierten Zellen.

Die Zellen können beispielsweise aus Gefäßfragmenten gewonnen werden, in dem die Gewebestückchen zunächst, wie in Zünd et al. (Eur. J. Cardiothorac. Surg. 13, 160, 1998) beschrieben, in Gewebebruchstücke zerstückelt und ca. 1 bis 2 Wochen unter normalen Zellkulturbedingungen (37°C, 5 CO₂, 95 % Luftfeuchtigkeit) inkubiert werden, bis die Zellen auf dem Boden der Kulturschale eine konfluente Zellschicht bilden. Anschließend werden sie mehrfachen Passagen unterworfen, um eine von restlichem Gewebematerial freie Zellkultur zu erhalten. Nach zwei bis drei Passagen können die gemischten Zellpopulationen separiert werden, in dem sie mit einem für Endothelzellen spezifischen Fluoreszenzmarker (Dil-Ac-LDL, von Medical Technologies Inc., Stoughton, MA) inkubiert werden und mittels Durchflußzytometrie (FACStar Plus, Becton Dickinson) getrennt werden. Fluoreszenzmarkierte Zellen sind hier Endothelzellen, nicht markierte Zellen sind Fibroblasten und Myofibroblasten. Diese werden weitere zwei bis drei Wochen kultiviert und während dieser Zeit zwei bis vier Passagen unterworfen, um eine ausreichende Anzahl an Zellen für die anschließende Besiedelung des Trägers zu erhalten. Eine weitere etablierte Methode zur Separation von gemischten Zellpopulationen stellt die Markierung einer Zellsorte mit schwach magnetisch geladenen spezifischen Antikörpern dar ("immunomagnetic beads"). Aufgrund des hier entstehenden spezifischen Magnetismus erfolgt die Trennung z.B. der Endothelzellen von den Myofibroblasten.

In den so erhaltenen Fibroblastenkulturen liegen Fibroblasten und Myofibroblasten gemischt vor. Das Verhältnis beider Populationen schwankt. Es ist bevorzugt, Kulturen zu verwenden, in denen der Fibroblastenanteil überwiegt; besonders bevorzugt ist ein Fibroblastenanteil von mehr als 75 %. Eine wie beschrieben gereinigte oder jede andere reine Fibroblasten/Myofibroblastenkultur kann nunmehr zur Besiedelung des Polymerträgers eingesetzt werden. Dazu werden pro Quadratzentimeter des Trägers ca. 10⁵ bis 5 x 10⁸ Fibroblasten und/oder Myofibroblasten, bevorzugt etwa 4 bis 5 x 10⁶, eingesetzt. Unter "Oberfläche" ist in diesem Fall nicht die tatsächliche Oberfläche des Polymers, sondern bei Herzklappen die bei Betrachtung des Trägers von oben in einer Ebene erkennbare Fläche und bei Gefäßen die nach Aufschneiden der Röhren und Ausbreiten auf einer Ebene in dieser Ebene erkennbare Fläche gemeint. Üblicherweise werden den Fibroblasten mindestens 60 bis 90 min Zeit gegeben, um sich an den Träger anzuheften. Anschließend kann das überstehende Medium entfernt werden und ein weiteres Mal Fibroblastensuspension zugegeben werden. Der Zeitraum zwischen der ersten und zweiten Zugabe von Fibroblastensuspension kann jedoch bis zu 36 Stunden, bevorzugt bis zu 24 Stunden für Herzklappenprothesen und für Gefäßprothesen betragen.

Das bei der Inkubation verwendete Medium kann jedes handelsübliche Zellkulturmedium sein; bevorzugt wird DMEM verwendet, dem wahlweise 5 oder 10 % fötales Kälberserum zugesetzt sein können. Zur Beschleunigung der Zellproliferation können Wachstumshormone (z.B. basic fibroblast growth factor (bFGF)) zugesetzt werden. Zur Stimulation der Bildung von Extrazellulärmatrix, insbesondere Kollagen, kann Vitamin C supplementiert werden.

In einer bevorzugten Ausführungsform des erfindungsgemäßen Verfahrens werden dem Träger bzw. sich der nach der erstmaligen Fibroblastenzugabe allmählich ausbildenden Matrix weitere 3 bis 14 mal, besonders bevorzugt 5 bis 10 mal Fibroblasten und/oder Myofibroblasten zugefügt.

Unter den üblicherweise für das Zellwachstum von Fibroblasten verwendeten Bedingungen (z. B. 5 % CO₂, Inkubation bei 37° C, steriles Medium) entwickelt sich nach ca. ein bis drei Wochen eine solide bindegewebige Struktur. Nunmehr wird diese Struktur mit einer reinen Endothelzell-Suspension besiedelt und inkubiert. Die Endothelzellen können genauso wie die Fibroblasten durch z.B. FACS angereichert und anschließend in mehreren Passagen (bevorzugt 3) expandiert werden. Auch für Endothelzellen ist es bevorzugt, die Besiedelung mit jeweils ca. 10⁵ bis 5 x 10⁸, bevorzugt 4 bis 5 x 10⁶, Endothelzellen mehrfach zu wiederholen, z.B. 3 bis 14 mal. In bevorzugten Ausführungsformen wird die Besiedelung mit Endothelzellen 5 bis 10 mal wiederholt. Zwischen zwei Besiedelungsschritten sollten mindestens 60-90 min, bevorzugt jedoch 2 bis 24 Stunden für Herzklappenprothesen und für Gefäßprothesen liegen.

Bei den zum Besiedeln des Trägers verwendeten Zellen handelt es sich um humane Zellen. Besonders bevorzugt ist es jedoch, autologe Fibroblasten und/oder Myofibroblasten sowie Endothelzellen und/oder Stammzellen zu verwenden. Dazu wird dem Patienten, dessen Herzklappe bzw. Blutgefäß ersetzt werden soll, Gewebe z.B. aus einem seiner Gefäße entnommen. Wie oben bereits erwähnt, bieten sich dazu die Arteria radialis sowie die Vena saphena an. Stammzellen können aus dem Knochenmark oder direkt aus dem Blut gewonnen werden. Die Verwendung der autologen Zellen zur Konstruktion von Herzklappen oder Gefäßen hat den wesentlichen Vorteil, daß weder die Klappe noch ein Gefäß nach Implantation in den Patienten körperfremdes Gewebe darstellt und somit Immunreaktionen gegen die künstliche Herzklappe oder das künstliche Gefäß so gut wie ausgeschlossen sind.
Ca. 14 Tage nach Zugabe der Endothelzellen läßt sich histologisch und immunhistochemisch ein Herzklappen-analoges bzw. ein Gefäß-analoges Gewebe mit einer superfiziellen konfluenten Einzelzellschicht aus Endothelzellen und einer bindegewebigen Grundstruktur nachweisen. Dieses Gewebe ist jedoch zur Implantation in den menschlichen Organismus nur bedingt geeignet, da es auf Grund seiner zu schwachen mechanischen Eigenschaften den dort herrschenden Strömungsverhältnissen nicht gewachsen wäre.

Erfindungsgemäß wird nunmehr in einem weiteren Verfahrensschritt die vorgebildete Herzklappen- bzw. Gefäß-analoge Struktur in ein pulsatiles System eingebracht, in dem sie steigenden Flußraten und/ oder Drücken ausgesetzt werden kann. Es wurde festgestellt, daß durch eine langsame Adaptation der Flußraten und Drücke die Bildung einer strömungsbeständigen Herzklappen- bzw. Gefäß-analogen Struktur erreicht werden kann.

Zum Durchführen des erfindungsgemäßen Verfahrens eignet sich ein Bioreaktor. Dieser Bioreaktor sollte möglichst kompakt ausgebildet sein, um in üblichen Zellinkubatoren eingesetzt werden zu können. Der Bioreaktor sollte eine Flußkammer aufweisen, in der im Fall der Herstellung einer Herzklappe ein Klappenventil angeordnet ist, und /oder ein verzweigtes Schlauchsystem, in den tubuläre Träger bzw. Matrices befestigt werden können. Der Antrieb zum Pumpen des Fluids durch die Flußkammer sollte dabei außerhalb der Flußkammer angeordnet sein. Dies wird bei dem verwendeten Bioreaktor durch eine der Flußkammer benachbarte Luftkammer erzielt, die durch eine hochelastische Membran von der Flußkammer getrennt ist. Durch pulsierendes Ändern des Luftdrucks in der Luftkammer kann ein Fördern des Fluids durch die Flußkammer erzeugt werden. Anstelle von Luft könnte auch eine Flüssigkeit oder ein anderes Gas in der Luftkammer aufgenommen sein, die dann allgemein auch als Antriebsmediumkammer bezeichnet werden kann. Dann müßte dieses Gas oder das darin befindliche Fluid jeweils pulsierenden Druckänderungen unterworfen werden. Luft läßt sich jedoch besonders leicht handhaben und hat sich daher als besonders geeignet für das Betreiben des Bioreaktors erwiesen.

Aufgrund der Luftkammer kann der Antrieb zum Fördern des Fluids in der Flußkammer sehr kompakt gestaltet werden und auch außerhalb des Inkubators angeordnet sein. Es läßt sich dadurch eine Trennung herbeiführen zwischen dem Teil, der unmittelbar auf die Flußkammer wirkt, und dem Teil, der die Druckschwankungen in der Luftkammer, bzw. Antriebsmediumkammer erzeugt. So kann z. B. eine Respiratorpumpe außerhalb des Inkubators angeordnet sein, die über einen dünnen Schlauch mit der Luftkammer, bzw. Antriebsmediumkammer verbunden ist Dadurch läßt sich die Gestaltung des Bioreaktors und der Antrieb selbst den jeweiligen Einbauerfordernissen anpassen.

Die für das erfindungsgemäße Verfahren verwendete pulsatile Flußkammer ist Bestandteil eines Bioreaktors. Es versteht sich, daß sowohl der Bioreaktor als auch die ihn umfassende Anordnung nicht nur im erfindungsgemäßen Verfahren, sondern auch davon unabhängig verwendet werden könnten.

Die folgenden Abbildungen und Beispiele erläutern die Erfindung:

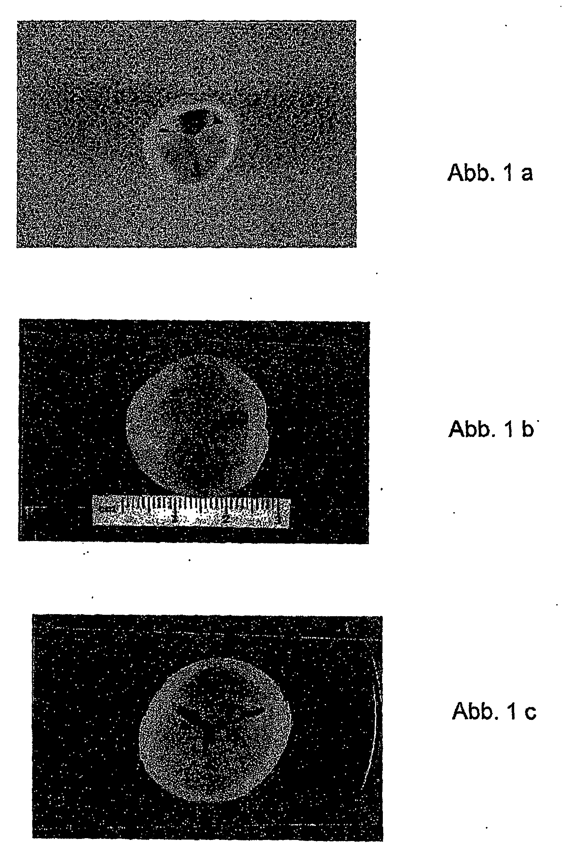

Abbildung 1 a zeigt einen aus einem Polymer vorgebildeten Träger für eine Herzklappenprothese, der anschließend einer Besiedlung mit Fibroblasten/Myofibroblasten und Endothelzellen unterworfen wird. Abbildung 1 b zeigt den Träger/die Matrix nach Besiedelung und einer Woche Inkubation in der pulsatilen Flußkammer, Abb 1 c zeigt die gleiche Klappenprothese nach 2 Wochen Inkubation in der Flußkammer.

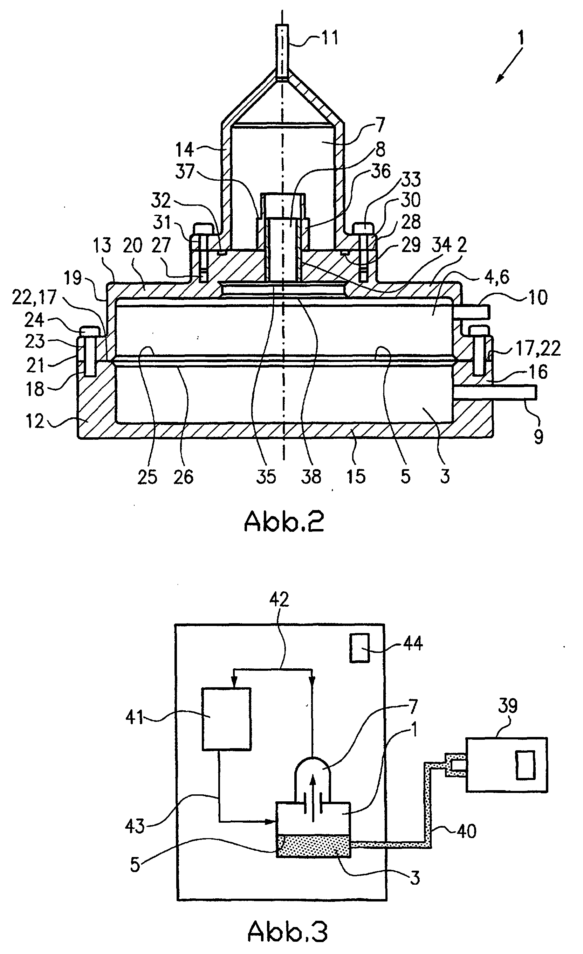

Abbildung 2 zeigt einen Bioreaktor einschließlich einer pulsatilen Flußkammer in Schnittansicht.
Abbildung 3 zeigt den Gesamtaufbau, in den der Bioreaktor integriert ist.

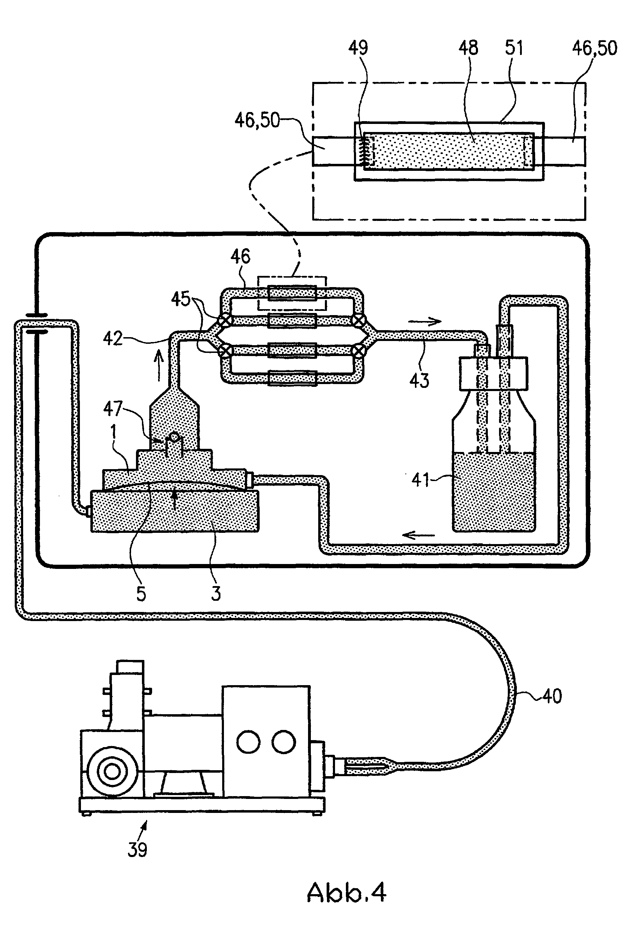

Abbildung 4 zeigt einen Gesamtaufbau mit integriertem parallel geschalteten Schlauchkreislaufsystem zum Herstellen von Gefäßprothesen.

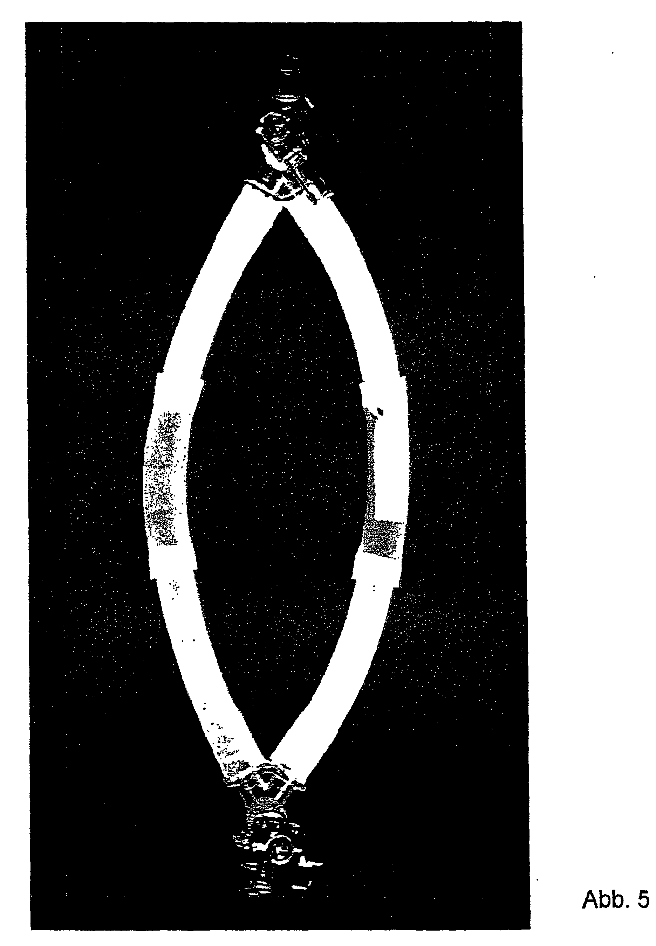

Abbildung 5 zeigt parallel geschaltete Schlauchkreislaufsysteme, wobei in einem der Schläuche ein gefäßförmiger Träger/Matrix eingenäht ist. Der Fluidstrom verläuft vom oberen (Naht-nahem) Y-Konnektor zum unteren.

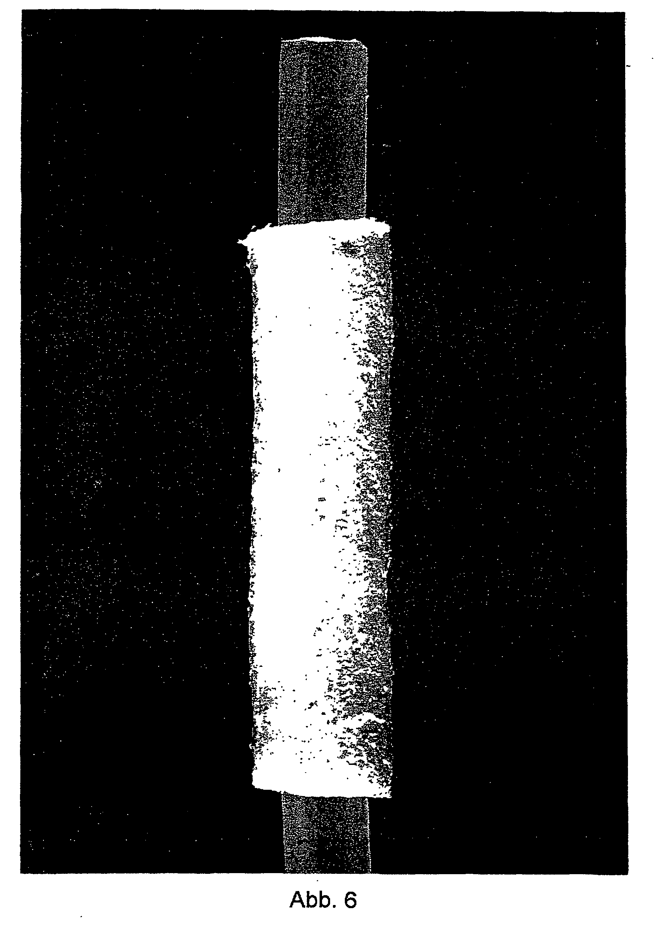

Abbildung 6 zeigt einen auf einen Zylinder aufgesteckten gefäßförmigen Träger vor der Besiedlung mit Zellen.

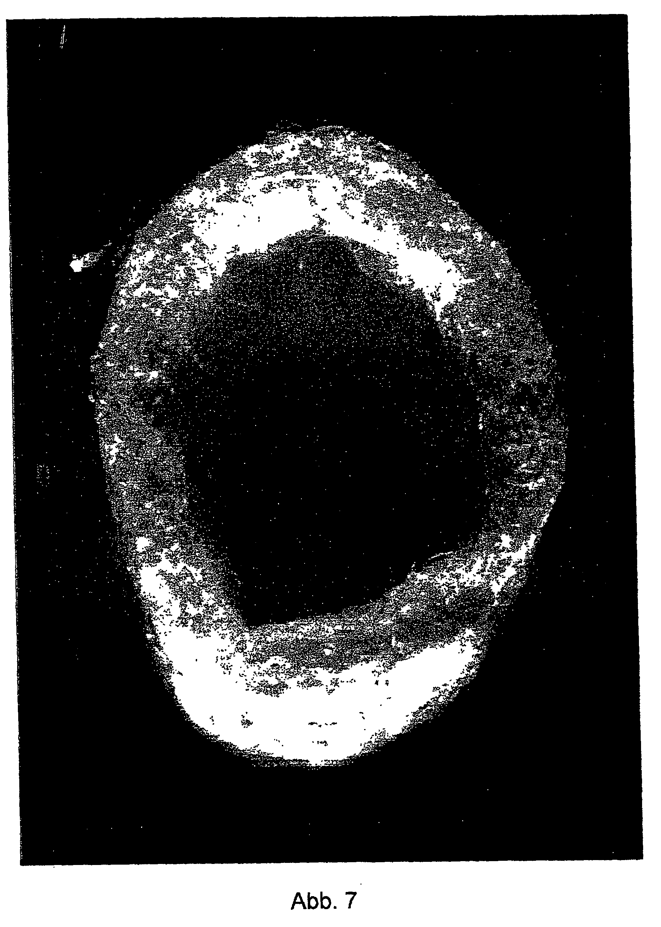

Abbildung 7 stellt einen Querschnitt durch den in Abbildung 6 gezeigten Träger dar. In Abb. 2 ist ein Bioreaktor 1 in einer Schnittansicht dargestellt. Der Bioreaktor ist um die in der Abb. 2 dargestellte vertikale Symmetrieachse im wesentlichen rotationssymmetrisch. Es handelt sich bei dem Bioreaktor um einen sogenannten Kompaktbioreaktor,: In einer bevorzugten Ausführungsform beträgt der Außendurchmesser in radialer Richtung ca. 15,5 cm und die Höhe ca. 16,8 cm. Abweichungen sowohl in der Höhe als auch des radialen Außendurchmessers können vorgenommen werden. Der Betrieb kann in Standard-Zellinkubatoren erfolgen.

Der Bioreaktor 1 verfügt über ein Gehäuse 2, mit zwei Kammern 3 und 4, die durch einen Membran 5 voneinander getrennt sind. Die untere Kammer 3 bildet eine Luftkammer und die obere Kammer 4 eine Flußkammer, die in zwei Teile unterteilt ist, wobei ein Teil durch einen Fluidkammerabschnitt 6 und der andere Teil durch einen Ventilperfusionskammerabschnitt 7 gebildet wird. Der Fluidkammerabschnitt 6 und der Ventilperfusionskammerabschnitt 7 sind über einen Durchgang 8 miteinander verbunden.

Die Kammern kommunizieren mit der Umgebung über Anschlüsse 9, 10 und 11, wobei Anschluß 9 in die Kammer 3, Anschluß 10 in den Fluidkammerabschnitt 6 der Flußkammer 4 und Anschluß 11 in den Ventilperfusionskammerabschnitt 7 der Flußkammer 4 mündet. Die Anschlüsse 9, 10 und 11 sind jeweils als über das Gehäuse vorstehende Rohrstutzen ausgebildet, auf die Schläuche oder Leitungen in bekannter Weise aufsteckbar sind.

Das Gehäuse ist dreiteilig ausgebildet, mit einem unteren Gehäuseteil 12, einem mittleren Gehäuseteil 13 und einem oberen Gehäuseteil 14. Das untere Gehäuseteil 12 ist im wesentlichen schalenförmig mit einem Boden 15 und einer im wesentlichen ringförmig verlaufenden Wand 16. Die der Wand 16 abgewandte Seite des Bodens bildet gleichzeitig die Auflagefläche für den Bioreaktor, mit der er z. B. auf einem Tisch aufgesetzt werden kann. Der Anschluß 9 erstreckt sich im wesentlichen radial aus der Wand 16 heraus. Die Wand 16 weist eine Flanschfläche 17 auf, in der sich in Axialrichtung erstreckende Gewindebohrungen 18 eingelassen sind. Um ein Beobachten der Vorgänge im Innern der Kammer 3 zu erleichtern, ist das untere Gehäuseteil aus durchsichtigem Material, z. B. Plexiglas (Polymethylmethacrylat, PMMA), hergestellt.

Das mittlere Gehäuseteil 13 ist ebenfalls im wesentlichen rotationssymmetrisch um die Symmetrieachse des Bioreaktors und verfügt über eine im wesentlichen zylindrische Wand 19, an die sich ein Deckelabschnitt 20 anschließt, sowie über einen Flansch 21 auf der dem Deckelabschnitt 20 abgewandten Seite der Wand 19. Der Flansch 21 ist im wesentlichen ringförmig mit einer der Flanschfläche 17 zugewandten Flanschfläche 22, die sich radial erstreckt. Im Flansch 21 sind zudem Durchgangsbohrungen 23 vorgesehen, denen jeweils fluchtend eine der Gewindebohrungen 18 zugeordnet ist Die Durchgangsbohrungen 23 und Gewindebohrungen 18 sind gleichmäßig am Umfang verteilt, wobei die bevorzugte Ausführungsform über neun Gewindebohrungen 18 mit zugehörigen Durchgangsbohrungen 23 verfügt.

Zwischen den beiden Flanschflächen 17 und 22 befindet sich die Membran 5. Durch die Membran 5 werden die beiden Kammern 3 und 4 voneinander getrennt. Die Membran besteht aus hochelastischem Silikon, das in der Art eines Trommelfells zwischen die Flanschflächen 17 und 22 gespannt ist. Die Membran weist eine Dicke von ca. 0,5 mm auf, und kann dabei so ausgebildet sein, daß im montierten Zustand ihr Außendurchmesser im wesentlichen dem Außendurchmesser des unteren und des mittleren Gehäuseteils entspricht. Dann muß die Membran mit Öffnungen im Bereich der Durchgangsbohrungen 23 versehen sein. Um die Membran 5 fest zwischen den Flanschflächen 17 und 22 einzuspannen, werden durch die Durchgangsbohrungen 23 Schrauben 24 aus rostfreiem Stahl in die Gewindebohrungen 18 eingeschraubt, die sich auch durch die Öffnungen der Membran hindurch erstrecken. Durch die Schrauben 24 werden die Flanschflächen 22 und 17 gegeneinander gedrückt, um dadurch die Membran fest einzuspannen und eine optimale Dichtheit zu erzielen.

Während des Betriebs des Bioreaktors schwingt die Membran in Axialrichtung. Um die Belastung der Membran am Übergang von der Flanschfläche 17 und 22 zu den zugeordneten Wänden 16 und 19 zu verringern, sind dort jeweils umlaufende Phasen 25 und 26 vorgesehen.

Der Fluidkammerabschnitt 6 wird somit nach unten durch die Membran 5 und nach oben durch den Deckelabschnitt 20 begrenzt. Um die Reaktionen in dem Fluidkammerabschnitt 6 visuell überwachen zu können, besteht das mittlere Gehäuseteil wie auch das untere Gehäuseteil aus durchsichtigem Material, z. B. Plexiglas.

An das mittlere Gehäuseteil 13 schließt sich das obere Gehäuseteil 14 an. Dieses ist im wesentlichen glockenförmig und über eine Flanschverbindung mit dem mittleren Gehäuseteil verbunden. Für diese Flanschverbindung verfügt das mittlere Gehäuseteil 13 in seinem Deckelabschnitt über sechs Gewindebohrungen 27, die sich in Axialrichtung erstrecken und gleichmäßig am Umfang verteilt sind. Die Gewindebohrungen 27 sind dabei in einer radial verlaufenden Flanschfläche 28 angeordnet, die eine umlaufende Nut 29 zur Aufnahme eines Dichtelements aufweist. Ein solches Dichtelement kann z. B. ein O-Ring sein. Die Nut 29 ist gegenüber den Gewindebohrungen 27 radial nach innen versetzt.

Das obere Gehäuseteil 14 verfügt über einen Flanschring 30 mit Durchgangsbohrungen 31 und einer der Flanschfläche 28 zugewandten Flanschfläche 32. Das obere Gehäuseteil 14 steht mit seiner Flanschfläche 32 auf der Flanschfläche 28 auf und ist über Schrauben 33 aus rostfreiem Stahl, die durch die Durchgangsbohrungen 31 gesteckt und in die Gewindebohrungen 27 jeweils eingeschraubt werden, mit dem mittleren Gehäuseteil 13 fest verbunden. Das Dichtelement wird dabei von der Flanschfläche 32 in die Nut 29 gedrückt und dichtet den Ventilperfusionskammerabschnitt 7 zur Umgebung hin ab. Der Anschluß 11 ist in der Spitze des oberen Gehäuseteils 14 angeordnet. Analog der beiden anderen Gehäuseteile 12 und 13 besteht das obere Gehäuseteil 14 aus durchsichtigem Material z. B. Plexiglas.

Wie bereits eingangs erwähnt, sind der Fluidkammerabschnitt 6 und der Ventilperfusionskammerabschnitt 7 über einen Durchgang 8 miteinander verbunden. Dieser Durchgang 8 wird durch eine Durchgangsbohrung 34 im Deckelabschnitt 20 des mittleren Gehäuseteils 13 gebildet. In diese Durchgangsbohrung 34, die sich axial erstreckt, ist ein Rohr 35 eingeschoben, das sich über den Deckelabschnitt 20 hinaus erstreckt. Auf den Rohrabschnitt des Rohrs 35, der sich über den Deckelabschnitt 20 des mittleren Gehäuseteils 13 hinaus erstreckt, ist ein Rohr 36 aufgesteckt. Dieses Rohr ist abnehmbar. Am Außendurchmesser des Rohrs ist eine ringförmige Stufe 37 vorgesehen. Durch die Gestaltung des Rohrs 36 und der Stufe 37 ist es möglich, Ventile auf das Rohr 36 aufzustecken. Auch ist es möglich, in das Rohr 36 Ventile oder Filter einzustecken, da das Rohr 35 einen kleineren Innendurchmesser als das Rohr 36 aufweist oder dadurch die Stirnseite des Rohrs 35 eine Anlagefläche für einen Filter oder dergleichen bilden kann. Für die erfindungsgemäße Herstellung von homologen Herzklappen ist das Ventil ein nicht näher dargestelltes 2-segeliges oder 3-segeliges Klappenventil, das dem oben beschriebenen Träger, bzw. der Matrix entspricht, und als Rückschlagventil arbeitet, um einen Fluidstrom nur von dem Fluidkammerabschnitt zu dem Ventilperfusionskammerabschnitt zu ermöglichen. Das Klappenkonstrukt des Klappenventils ist auf einen Ring eingenäht. Zum Befestigen des Klappenventils am Rohr 36 wird der Ring auf das Rohr 36 gesteckt und durch Reibschluß gehalten. Sowohl das Rohr 36 als auch der Ring bestehen bevorzugt aus Silikon, die Verwendung anderer Materialien ist jedoch nicht ausgeschlossen.

An der Unterseite des Deckelabschnitts ist eine ringförmige Vertiefung 38 vorgesehen, die konzentrisch zum Rohr 35 verläuft und in die das Rohr 35 mündet. Diese Vertiefung 38 kann z. B. als Aufnahme für Filtermaterialien oder dergleichen dienen.

Im weiteren wird eine Bioreaktoranordnung mit dem oben beschriebenen Bioreaktor 1 beschrieben. Zum Betreiben des Bioreaktors ist eine Respiratorpumpe 39 vorgesehen, die über einen Schlauch 40 und den Anschluß 9 mit der Kammer 3 verbunden ist. Die Respiratorpumpe erzeugt einstellbare Druckimpulse, durch die der Druck der Kammer periodisch erhöht werden kann. Die Respiratorpumpe ist eine gesteuerte Zweiphasen-Respiratorpumpe, die als Luftpumpe funktioniert. Mit der Pumpe kann das Pumpvolumen und die Pumpfrequenz eingestellt werden, wobei der Fluß in einem Bereich von 50 ml pro Minute bis 5000 ml pro Minute liegt, und der Systemdruck zwischen 20 und 240 mmHg variieren kann.

Ein Reservoir 41 ist über Schläuche 42 und 43 jeweils mit den Anschlüssen 11 und 10 verbunden. Durch die Schläuche 42 und 43 und das Reservoir 41 entsteht ein Kreislauf, wobei Fluid aus den Ventilperfusionskammerabschnitt 7 über den Anschluß 11, den Schlauch 42 in das Reservoir 41 und von dort aus über den Schlauch 43 und den Anschluß 10 in den Fluidkammerabschnitt 6 gefördert werden kann. Die Schläuche 42 und 43 sind bevorzugt Silikonschläuche.

In einer weiteren Ausführungsform wird das Schlauchsystem 42, welches der Perfusionskammer 1 nachgeschaltet ist, und durch welches das Nährmedium zum Reservoir 41 zurückfließt, über Y-Konnektoren 45 auf 2, 4, oder mehr parallelgeschaltete Schlauchkreislaufsysteme 46 erweitert. Um einen gerichteten pulsatilen Fluidstrom durch dieses System zu ermöglichen, wird in den Ventilperfusionskammerabschnitt 7 ein synthetisches Rückschlagventil 47 installiert. In jedes der parallelgeschalteten Schlauchkreislaufsysteme kann ein mit Zellen besiedeltes röhrenförmiges Polymer als Träger/ Matrix 48 eingebracht werden (siehe Abb. 4). Hierbei wird der besiedelte gefäßförmige Träger/ die Matrix 48 unter sterilen Bedingungen, z.B. mittels einer Naht 49 oder einer anderen Verbindungseinrichtung an dem proximalen Schlauchende 50 so angeschlossen, dass das Nährmedium durch das Lumen des Schlauchendes 50 in den gefäßförmigen besiedelten Träger/die Matrix 48 strömt und diesen/diese pulsatil durchströmt. Umgeben wird der an das Schlauchsystem 42/46 angeschlossene Träger/ die Matrix 48 von einem weiteren Schlauch 51, der in seinen Dimensionen (innerer Durchmesser) so gewählt ist, dass er jeweils über die Schlauchenden 50 gestülpt werden kann und eine dichte Umschließung bildet. In diesem Schlauchsystem werden die besiedelten Träger/ Matrices 48, welche verschiedene Durchmesser aufweisen können, verschiedenen pulsatilen Flüssen und systemischen Drücken ausgesetzt. Sowohl des Schlauchkreislaufsystem 46 als auch der weitere Schlauch 51 bestehen ebenfalls bevorzugt aus Silikon. Ersatzweise können sterilisierte Schlauchmaterialien mit gleichwertiger Flexibilität und Haltbarkeit verwendet werden.

Der Bioreaktor 1 und das Reservoir 41 mit den Schläuchen 42 und 43 befinden sich in einem standardisierten Inkubator 44, in dem bevorzugt Bedingungen von 37°C und 5% CO₂ gegeben sind.

Nachfolgend wird die Wirkungs- und Funktionsweise des Bioreaktors näher erläutert:

Über die Respiratorpumpe 39 werden Druckimpulse in die Kammer 3 geleitet Aufgrund dieser Druckimpulse dehnt sich die Membran 5 aus, wobei sie sich in der Darstellung in Abb. 2 mit jedem Druckimpuls nach oben wölbt und dabei auch zu einem Druckanstieg in dem Fluidkammerabschnitt 6 der Flußkammer 4 führt. Dieser Druckanstieg wird in den Durchgang 8 weitergeleitet und öffnet dadurch das nicht näher dargestellte Klappenventil am (Silikon-)Rohr 36. Auf diese Weise wird das im Fluidkammerabschnitt 6 vorhandene Fluid in den Ventilperfusionskammerabschnitt 7 gefördert. Aus den Ventilperfusionskammerabschnitt 7 wird Fluid dann in Pulswellen über den Anschluß 11 und den Schlauch 42 unmittelbar oder über das Schlauchkreislaufsystem 46 in das Reservoir 41 gefördert. Von dort aus kann es über den Schlauch 43 in den Fluidkammerabschnitt 6 zurückgefördert werden. Bei abfallendem Druck in der Kammer 3 wird die Membran 5 wieder aufgrund ihrer Eigenspannung in ihre Ausgangsstellung rücküberführt, in welcher sie sich im wesentlichen radial erstreckt. Durch den Druckabfall erfolgt auch in dem Fluidkammerabschnitt 6 ein Druckabfall, der wiederum zu einem Schließen des als Klappenventils ausgebildeten Ventils führt. Der Druckabfall pflanzt sich bis zum Schlauchkreislaufsystem 46 fort.

Durch den pulsierenden Druckanstieg in der Kammer bzw. im Schlauchkreislaufsystem kann somit ein Kreislauf erzeugt werden, der im wesentlichen die physiologischen Flußbedingungen im Herzen simuliert. Mit dem neuartigen Bioreaktor wird eine hohe Dichtigkeit des Fluidkammerabschnitts 6 und des Ventilperfusionskammerabschnitts 7 erreicht. Aufgrund des dadurch möglichen Schutzes vor Infektionen können lange Kultivierzeiten ermöglicht werden. Durch den Antrieb mit Luft über die Kammer 3, die durch die Membran 5 hermetisch von der Fluidkammer 6 getrennt ist, lassen sich die Probleme von Hitzeentwicklungen innerhalb des Inkubators 44 vermeiden, da der eigentliche Pumpmotor (Respiratorpumpe) sich außerhalb des Zellinkubators befindet.

Da der gesamte Bioreaktor transparent ist, kann man die Klappenkonstruktion permanent einsehen und das Öffnen und Schließen der Klappen kontrollieren. Weiterhin kann man an der Färbung der Nährflüssigkeit pH-Veränderungen erkennen. Das Reservoir mit dem Fluid kann durch sterile Konnektoren an die Schläuche angeschlossen werden. Dadurch läßt sich ein sicherer Fluidwechsel ermöglichen.

Aufgrund der einfachen Konstruktion des Bioreaktors kann ein Austausch der Ventile bzw. der Herzklappen auf einfache Weise durchgeführt werden. Dazu müssen nur die Schrauben 33 gelöst und das obere Gehäuseteil 14 abgenommen werden. Das Ventil bzw. die Herzklappe kann dann ausgetauscht werden und anschließend kann das obere Gehäuseteil 14 durch die Schrauben 33 wieder am mittleren Gehäuseteil befestigt werden.

Ebenso können Gefäßprothesen leicht aus dem Schlauchkreislaufsystem 46 entnommen werden. Dazu wird bei abgeschalteter Pumpe 39 der äußere Schlauch 51 entfernt, die proximale Anastomose (Näht) 49 steril aufgetrennt und die Gefäßprothese steril entnommen.

In der Ausführungsform der Erfindung werden Flußraten zwischen 50 ml/min und 5000 ml/min, bevorzugt 50 ml/min bis 2000 ml/min verwendet. Die Angaben beziehen sich auf den Fluß durch die Klappenprothese bzw. Gefäßprothese. Als anfängliche Flußrate haben sich minimale Flußraten von 50 bis 100 ml/min als geeignet erwiesen. Diese Flußraten werden z.B. mit einer Pulsfrequenz von 5 bis 20 Pulsen pro Minute, bevorzugt 5 - 10 Pulsen/min, durch die Prothese geschickt. Die Flußrate wird anschließend kontinuierlich oder diskontinuierlich auf bis zu 5000 ml/min gesteigert. Gleichzeitig wird die Pulsfrequenz auf bis zu 180 Pulse/min angehoben. Bei den angegebenen Daten handelt es sich um die Grenzwerte, die normalerweise nicht überschritten werden.

In bevorzugten Ausführungsformen wird die Flußrate bis auf 2000 ml/min gesteigert, während die Pulsfrequenz auf 60 bis 100, bevorzugt 80 Pulse/min angehoben wird. Die Belastung der sich stabilisierenden Herzklappe bzw. der Gefäßprothese wird damit nahezu physiologischen Verhältnissen angepaßt. Es hat sich als günstig, aber nicht notwendig erwiesen, die Flußrate und die Pulsfrequenz jeweils nach ca. 24 bis 48 Stunden zu steigern. So kann beispielsweise, ausgehend von einer Flußrate von 50 bis 100 ml/min und einer Pulsrate von 5 bis 10 Pulsen/min am Tag 1 des Aufenthaltes in der pulsatilen Flußkammer bzw. des Schlauchkreislaufsystems 46, am Tag 3 eine Erhöhung auf 300 ml/min bei 20 bis 25 Pulsen/min, am Tag 5 auf 700 ml/min und 35 bis 45 Pulsen/min, am Tag 7 auf 1000 ml/min und 50 bis 60 Pulsen/min, am Tag 9 auf 1300 ml/min und 70 bis 80 Pulsen/min, am Tag 11 auf 1500 ml/min und ca. 100 Pulsen/min, am Tag 13 auf 1750 ml/min und ca. 120 Pulsen/min und am Tag 15 auf 2000 ml/min und 140 Pulsen/min vorgesehen werden. Je nach zur Verfügung stehender Zeit, Größe der Klappe, bzw. Durchmesser und Länge der Gefäßprothese, Größe und Alter des Patienten etc. kann jedoch eine sehr viel langsamere Steigerung der Flußraten sowie der Pulsfrequenz oder die Steigerung auf höhere Flußraten und Pulsfrequenzen sinnvoll sein. Gute Erfahrungen wurden mit folgenden Steigerungschemata gemacht:

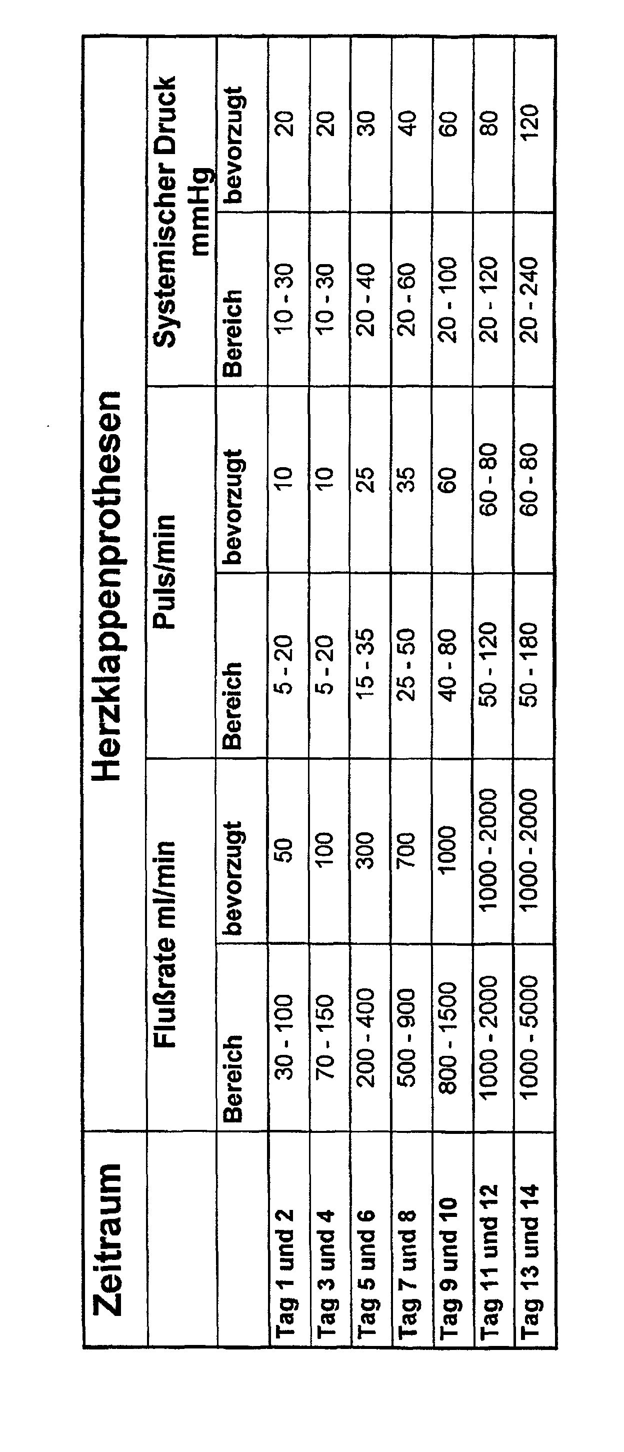

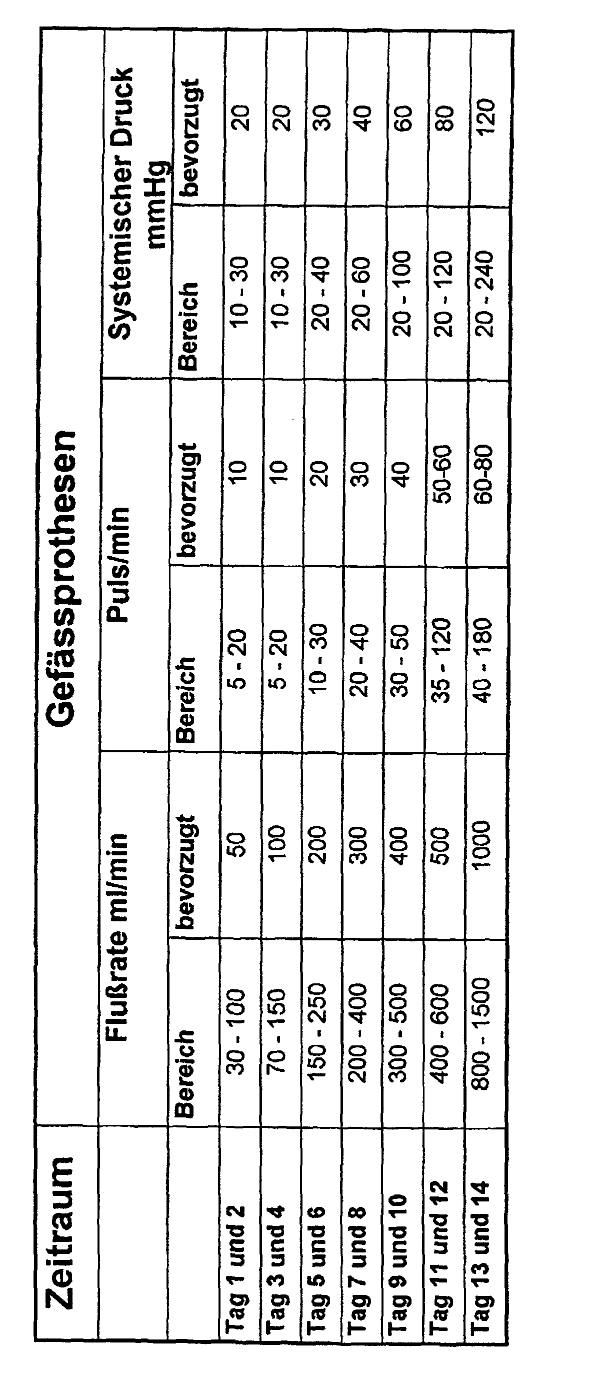

In der Ausführungsform der Erfindung wird der in der pulsatilen Flußkammer herrschende systemischen Druck kontinuierlich oder diskontinuierlich von 10 mmHg bis auf 120-240 mmHg erhöht. Bevorzugt ist ein systemischer Druck bis 140, besonders bevorzugt bis 120 mmHg erhöht.

Die mittels des erfindungsgemäßen Verfahrens hergestellten homologen bzw. autologen Herzklappen und Gefäßprothesen weisen gegenüber den herkömmlichen mechanischen und biologischen Herzklappen und Gefäßprothesen wesentliche Vorteile auf. So bestehen sie in ihrer bevorzugten Ausführungsform aus autologem Gewebe, d.h. aus Gewebe des zur Operation anstehenden Patienten, und vermeiden dadurch jede Fremdkörperreaktion des Implantatempfängers. Die Infektionsgefahr bei Empfängern autologer Herzklappen unterscheidet sich nicht von der eines gesunden Herzens. Eine Antikoagolationstherapie ist nicht erforderlich; damit entfällt die Gefahr hemorrhagischer Komplikationen. Der bei weitem überzeugendste Vorteil der erfindungsgemäßen Herzklappen und der Gefäßprothesen ist jedoch die Tatsache, daß sie lebendes Gewebe darstellen und daher nach Implantation bei Wachstum des Empfängers mitwachsen. Dieser Vorteil ist insbesondere bei Herzklappen für kindliche Empfänger von großer Bedeutung. Das macht die erfindungsgemäße Herzklappe zur Klappe der Wahl bei Kindern und jugendlichen Patienten, deren Herz sich entwicklungsbedingt noch vergrößert. Die lebende Herzklappe wächst entsprechend mit, so daß auch bei Veränderungen des Herzens keine Dysproportionen (z. B. Verengungen) zwischen Klappe und Herz auftreten.

Die erfindungsgemäßen Herzklappen und Gefäßprothesen enthalten eine bindegewebige innere Struktur, die neben Fibroblasten und Myofibroblasten im wesentlichen Bestandteile einer normalen extrazellulären Matrix, nämlich Kollagen, Elastin und Glycosaminoglykane enthält. Im Gegensatz zu früheren Versuchen, durch "Tissue Engineering" z.B. Herzklappengewebe herzustellen, weisen die erfindungsgemäßen Klappen einen der nativen Klappe bzw. dem nativen Klappensegel entsprechenden Anteil an Kollagen (43 - 55 %), Elastin (11 - 13 %) und Glycosaminoglycan auf.

Es konnte gezeigt werden, daß die erfindungsgemäßen Herzklappen Flußraten von mehr als 3000 ml/min, entsprechend den in einem erwachsenen Humanherzen herrschenden Flußverhältnissen, standhalten. Somit kann erfindungsgemäß erstmals eine autologe Herzklappe zur Verfügung gestellt werden, die bedingungslos zur Implantation in kindliche wie auch erwachsene Patienten geeignet ist.

### Beispiel 1: Herstellung von Trägern

Zum Herstellen des herzklappenförmigen Trägers wird ein nicht-gewebtes Polyglycolsäurepolymer (Fiberdurchmesser: 12 - 15 µm, Polymerdichte: 70 mg/ml, Albany International Research, Mansfield MA, USA.) verwendet. Das Polymer wird in der Art zugeschnitten, daß es eine Röhre mit 19 mm Durchmesser bildet. In diesen Conduit werden 3 dreieckige Segel eingenäht. Dieser Träger kann zum Herstellen von 3-segeligen Klappen, d. h. Pulmonal- Aorten- und Tricuspidalklappen verwendet werden. Für Mitralklappen werden 2 Segel eingenäht.

Zum Herstellen eines gefäßförmigen Trägers (innerer Durchmesser z.B. 0,5 cm) wird ein Mischpolymer aus PGA 90% und P4HB 10 % verwendet. Das Polymer wird um einen Zylinder (Durchmesser 0,5 cm) gewickelt und mit einer Überlappungszone von ca. 0,1 cm unter Applikation von Hitze (60-70°C) verschweisst. Durch die Wahl verschieden dicker Zylinder können gefäßförmige Träger mit verschiedenen Innendurchmessern auf diese Weise hergestellt werden.

### Beispiel 2: Herstellung einer dreisegeligen Herzklappenprothese

Ein dreisegeliger Träger wird sterilisiert und in Medium (DMEM, GIBCO BRL-Life Technologies) für 24 Stunden eingelegt, um die Polymeroberfläche einzuweichen. Daraufhin wird der klappenförmige Träger pro Quadratzentimeter Oberfläche mit 4 Mio. Fibroblasten alle 90 Minuten insgesamt 6 mal besiedelt. Weiterhin wird der besiedelte Träger für 2 Wochen inkubiert (5 % CO₂, 37 ° C, 95 % Luftfeuchtigkeit). Das Medium wird alle 4 Tage unter sterilen Bedingungen gewechselt. Anschließend werden Endothelzellen auf den besiedelten klappenförmigen Träger aufgebracht (3 - 4 Mio. Endothelzellen pro Quadratzentimeter Oberfläche, 6 Besiedlungen alle 90 Minuten). Nach weiteren 2 Wochen wird das entstandene Gewebe in die Flußkammer der Bioreaktors unter sterilen Kautelen eingebracht und hier mittels des Silikonringes in Durchflußposition installiert. Anschließend wird der Bioreaktor mit Medium gefüllt und in den Zellinkubator gestellt Nachdem über den Druckluftschlauch die Konnektion zur außerhalb des Inkubators stehenden Pumpe hergestellt wurde, wird mit minimalen pulsatilen Flüssen begonnen (50 ml/min). In 2 Tages-Schritten wird die Flußrate und Pulsrate gesteigert auf 100 ml/min (Puls 10), 300 ml (Puls 25), 700 ml (Puls 35), 1000 ml (Puls 60) für insgesamt weiter 4 Tage. Anschließend (nach 14 Tagen) wird das nun gebildete Gewebe unter sterilen Bedingungen entnommen und zur biochemischen, histologischen und mechanischen Analyse asserviert.

### Beispiel 3: Herstellung einer Gefäßprothese

Für die Herstellung eines kleinkalibrigen Gefäßes (z.B. als Ersatz für die Halsschlagader) mit einem Innendurchmesser von 0,5 cm werden 4 gefäßförmige Träger (Länge 4 cm, Innendurchmesser 0,5 cm) sterilisiert und für 24 h in Standard Medium (DMEM, Gibco BRL-LifeTechnologies) eingelegt, um die Polymeroberfläche einzuweichen. Daraufhin wird jeder gefäßförmige Träger pro Quadratzentimeter Oberfläche mit 4 - 5 Mio. Myofibroblasten (Fibroblasten und Myoblasten) alle 90 Minuten insgesamt 6 mal besiedelt. Dann werden die besiedelten Träger für 2 Wochen in einem Standard Zell-Inkubator inkubiert. Das Nährmedium wird alle 4 Tage unter sterilen Bedingungen gewechselt. Anschließend werden Endothelzellen auf die Innenseiten der besiedelten gefäßförmigen Träger aufgebracht (4 Mio. Endothelzellen pro Quadratzentimeter Oberfläche, 6 Besiedlungen alle 90 Minuten). Nach weiteren 4 - 5 Tagen werden die entstandenen röhrenförmigen Gewebe unter sterilen Kautelen in das Schlauchsystem des Bioreaktors eingebracht und in Durchflussposition installiert. In 48-Stunden Schritten werden die Flussrate und die Pulsrate gesteigert von initial 50 ml/min, Puls 10 auf 100 ml/min Puls 60 (100 ml/ Puls 10; 200 ml/ Puls 20; 300 ml/ Puls 30; 400 ml/ Puls 40; 500 ml/ Puls 50; 1000 ml/ Puls 60). Anschliessend wird nach insgesamt 14 Tagen das neu gebildete Gewebe entnommen und zur biochemischen, feingeweblichen und mechanischen Untersuchung asserviert.

## Patentansprüche

1. *In-vitro*-Verfahren zum Herstellen einer homologen Herzklappen- oder Gefäßprothese zur Implantation beim menschlichen Patienten, umfassend die folgenden Schritte:
- Bereitstellen eines biologisch abbaubaren Trägers,
- Besiedeln des Trägers mit homologen Fibroblasten und/oder Myofibroblasten zur Ausbildung einer bindegewebigen Matrix,
- Besiedeln des Trägers/der Matrix mit Endothelzellen,
- Einbringen des Trägers/der Matrix in eine pulsatile Flusskammer, in der er/sie steigenden Flussraten und/oder Druck ausgesetzt werden kann, und
- kontinuierliches oder diskontinuierliches Erhöhen der Flussrate von einem Anfangswert von 50-100 ml/min bis zu einem Endwert von 2000 - 5000 ml/min und/oder des Druckes auf 120 - 240 mm/Hg.

2. Verfahren nach Anspruch 1, **dadurch gekennzeichnet, daß** der biologisch abbaubare Träger eine biologisch abbaubare Polymermatrix oder eine azelluläre biologische Matrix ist.

3. Verfahren nach einem der Ansprüche 1 oder 2, **dadurch gekennzeichnet, daß** der Träger eine Polyglycolsäure (PGA), Polymilchsäure (PLA), Polyhydroxyalkanoat (PHA), Poly-4-Hydroxybutyrat (P4HB) oder eine Mischung aus zwei oder mehreren dieser Polymere umfaßt.

4. Verfahren nach einem der Ansprüche 1 bis 3, **dadurch gekennzeichnet, daß** der biologisch abbaubare Träger in Form einer Herzklappe vorgebildet ist.

5. Verfahren nach einem der Ansprüche 1 bis 3, **dadurch gekennzeichnet, dass** der biologische abbaubare Träger in Form eines Blutgefäßes vorgebildet ist.

6. Verfahren nach einem der Ansprüche 1 bis 4, **dadurch gekennzeichnet, daß** der Träger eine Polymerdichte von 40 bis 120 mg/cm³, bevorzugt 50 bis 80 mg/cm³ aufweist.

7. Verfahren nach einem der Ansprüche 1 bis 6, **dadurch gekennzeichnet, daß** der Träger ein poröses Polymer mit einer Porengröße von 80 bis 240 µm ist.

8. Verfahren nach einem der Ansprüche 1 bis 7, **dadurch gekennzeichnet, daß** die Fasern des Trägers einen Durchmesser von 6 bis 20 µm, bevorzugt 10 bis 18 µm aufweisen.

9. Verfahren nach einem der Ansprüche 1 oder 2, **dadurch gekennzeichnet, daß** der Träger ein Bindegewebsgerüst einer Tier-Herzklappe ist.

10. Verfahren nach einem der Ansprüche 1 oder 2, **dadurch gekennzeichnet**, das der Träger ein Bindegewebsgerüst eines Tier-Gefäßes ist.

11. Verfahren nach einem der Ansprüche 1 bis 10, **dadurch gekennzeichnet, daß** der Schritt des Besiedelns mit Fibroblasten und/oder Myofibroblasten 3 bis 14 mal, bevorzugt 5 bis 10 mal wiederholt wird.

12. Verfahren nach einem der Ansprüche 1 bis 11, **dadurch gekennzeichnet, daß** pro Quadratzentimeter Träger/Matrix und Besiedelungsschritt ca. 10⁵ bis 5 x 10⁸, bevorzugt 4-5 x 10⁶ Fibroblasten und/oder Myofibroblasten eingesetzt werden.

13. Verfahren nach einem der Ansprüche 1 bis 12, **dadurch gekennzeichnet, daß** der Schritt des Besiedelns mit Endothelzellen 3 bis 14 mal, bevorzugt 5 bis 10 mal wiederholt wird.

14. Verfahren nach einem der Ansprüche 1 bis 11, **dadurch gekennzeichnet, daß** pro Quadratzentimeter Träger/Matrix und Besiedelungsschritt ca. 10⁵ bis 5 x 10⁸, bevorzugt 4-5 x 10⁶ Endothelzellen eingesetzt werden.

15. Verfahren nach einem der Ansprüche 1 bis 14, **dadurch gekennzeichnet, daß** die Fibroblasten und/oder Myofibroblasten und/oder Endothelzellen humane Zellen sind.

16. Verfahren nach einem der Ansprüche 1 bis 15, **dadurch gekennzeichnet, daß** die Fibroblasten und/oder Myofibroblasten und/oder Endothelzellen autologe Zellen sind.

17. Verfahren nach einem der Ansprüche 1 bis 16, **dadurch gekennzeichnet, daß** in der pulsatilen Flußkammer Flußraten von 50 ml/min bis 5000 ml/min, bevorzugt 50 bis 2000 ml/min eingestellt werden.

18. Verfahren nach einem der Ansprüche 1 bis 17, **dadurch gekennzeichnet, daß** die Flußrate über einen Zeitraum von 1 Woche bis 12 Wochen gesteigert wird.

19. Verfahren nach einem der Ansprüche 1 bis 18, **dadurch gekennzeichnet, daß** die anfängliche Flußrate 50 bis 100 ml/min beträgt.

20. Verfahren nach einem der Ansprüche 1 bis 19, **dadurch gekennzeichnet, daß** die anfängliche Pulsfrequenz 5 bis 10 Pulse/min beträgt.

21. Verfahren nach einem der Ansprüche 1 bis 19, **dadurch gekennzeichnet, daß** die Flußrate bis auf max. 5000 ml/min für Gefäßprothesen und für Herzklappenprothesen gesteigert wird.

22. Verfahren nach einem der Ansprüche 1 bis 20, **dadurch gekennzeichnet, daß** die Pulsfrequenz bis auf max. 180 Pulselmin für Gefäßprothesen und für Herzklappenprothesen gesteigert wird.

23. Verfahren nach einem der Ansprüche 1 bis 17, **dadurch gekennzeichnet, daß** in der pulsatilen Flußkammer systemische Drücke von 10 bis 240 mmHg eingestellt werden.

24. Homologe Herzklappe, herstellbar mit dem Verfahren nach einem der Ansprüche 1 bis 23.

25. Homologe Herzklappe nach Anspruch 24 zur Implantation bei einem menschlichen Patienten, wobei die homologe Herzklappe einen bindegewebigen inneren Kern hat, der von einer Endothelzellschicht umgeben ist, und in dem bindegewebigen inneren Kern eine Kollagendichte von 43 bis 55 % gegeben ist.

26. Homologe Herzklappe nach Anspruch 24, **dadurch gekennzeichnet, dass** sie den Strömungsbedingungen im menschlichen Herzen standhält.

27. Homologes Gefäß, herstellbar mit dem Verfahren nach einer der Ansprüche 1 bis 23.

## Claims

1. *In vitro* process for the production of a homologous heart valve prosthesis or vessel prosthesis for implantation in human patients, comprising the following steps:
- provision of a biodegradable carrier,
- colonization of the carrier with homologous fibroblasts and/or myofibroblasts to develop a connective tissue matrix,
- colonization of the carrier/matrix with endothelial cells,
- introduction of the carrier/matrix into a pulsatile flow chamber in which it can be exposed to increasing flow rates and/or pressure, and
- continuous or discontinuous increasing of the flow rate from an initial value of 50-100 ml/min up to a final value of 2,000-5,000 ml/min and/or of the pressure to 120-240 mm/Hg.

2. Process according to claim 1, **characterized in that** the biodegradable carrier is a biodegradable polymer matrix or an acellular biological matrix.

3. Process according to one of claims 1 or 2, **characterized in that** the carrier comprises a polyglycollic acid (PGA), polylactic acid (PLA), polyhydroxyalkanoate (PHA), poly-4-hydroxybutyrate (P4HB) or a mixture of two or more of these polymers.

4. Process according to one of claims 1 to 3, **characterized in that** the biodegradable carrier is preformed in the form of a heart valve.

5. Process according to one of claims 1 to 3, **characterized in that** the biodegradable carrier is preformed in the form of a blood vessel.

6. Process according to one of claims 1 to 4, **characterized in that** the carrier has a polymer density of 40 to 120 mg/cm³, preferably 50 to 80 mg/cm³.

7. Process according to one of claims 1 to 6, **characterized in that** the carrier is a porous polymer with a pore size of 80 to 240 µm.

8. Process according to one of claims 1 to 7, **characterized in that** the fibres of the carrier have a diameter of 6 to 20 µm, preferably 10 to 18 µm.

9. Process according to one of claims 1 or 2, **characterized in that** the carrier is a connective tissue skeleton of an animal heart valve.

10. Process according to one of claims 1 or 2, **characterized in that** the carrier is a connective tissue skeleton of an animal vessel.

11. Process according to one of claims 1 to 10, **characterized in that** the step of colonization with fibroblasts and/or myofibroblasts is repeated 3 to 14 times, preferably 5 to 10 times.

12. Process according to one of claims 1 to 11, **characterized in that** approx. 10⁵ to 5 x 10⁸, preferably 4-5 x 10⁶ fibroblasts and/or myofibroblasts are employed per square centimetre of carrier/matrix and colonization step.

13. Process according to one of claims 1 to 12, **characterized in that** the step of colonization with endothelial cells is repeated 3 to 14 times, preferably 5 to 10 times.

14. Process according to one of claims 1 to 11, **characterized in that** approx. 10⁵ to 5 x 10⁸, preferably 4-5 x 10⁶ endothelial cells are employed per square centimetre of carrier/matrix and colonization step.

15. Process according to one of claims 1 to 14, **characterized in that** the fibroblasts and/or myofibroblasts and/or endothelial cells are human cells.

16. Process according to one of claims 1 to 15, **characterized in that** the fibroblasts and/or myofibroblasts and/or endothelial cells are autologous cells.

17. Process according to one of claims 1 to 16, **characterized in that** flow rates of 50 ml/min to 5,000 ml/min, preferably 50 to 2,000 ml/min, are established in the pulsatile flow chamber.

18. Process according to one of claims 1 to 17, **characterized in that** the flow rate is increased over a period of time of 1 week to 12 weeks.

19. Process according to one of claims 1 to 18, **characterized in that** the initial flow rate is 50 to 100 ml/min.

20. Process according to one of claims 1 to 19, **characterized in that** the initial pulse frequency is 5 to 10 pulses/min.

21. Process according to one of claims 1 to 19, **characterized in that** the flow rate is increased up to max. 5,000 ml/min for vessel prostheses and for heart valve prostheses.

22. Process according to one of claims 1 to 20, **characterized in that** the pulse frequency is increased up to max. 180 pulses/min for vessel prostheses and for heart valve prostheses.

23. Process according to one of claims 1 to 17, **characterized in that** systemic pressures of 10 to 240 mm Hg are established in the pulsatile flow chamber.

24. Homologous heart valve which can be produced by the process according to one of claims 1 to 23.

25. Homologous heart valve according to claim 24 for implantation in a human patient, wherein the homologous heart valve has a connective tissue inner core surrounded by an endothelial cell layer, and a collagen density of 43 to 55% exists in the connective tissue inner core.

26. Homologous heart valve according to claim 24, **characterized in that** it withstands the flow conditions in the human heart.

27. Homologous vessel which can be produced with the process according to one of claims 1 to 23.

## Revendications

1. Procédé *in vitro* de préparation d'une prothèse homologue de valvule cardiaque ou de vaisseau destinée à être implantée chez des patients humains, comportant les phases suivantes :
- production d'un support biologiquement dégradable,
- colonisation du support avec des fibroblastes et/ou des myofibroblastes homologues pour constituer une matrice de tissu conjonctif,
- peuplement du support/matrice avec des cellules endothéliales,
- introduction du support/matrice dans une chambre à débit pulsatile, dans laquelle on peut produire des débits qui augmentent et/ou une pression croissante, et
- augmentation continue ou par étapes du débit en partant d'un débit initial de 50-100 ml/min jusqu'à une valeur finale de 2000-5000 ml/min et/ou de la pression à 120-240 mm/Hg.

2. Procédé selon la revendication 1, **caractérisé en ce que** le support biologiquement dégradable est une matrice de polymère biologiquement dégradable ou une matrice biologique acellulaire.

3. Procédé selon l'une des revendications 1 ou 2, **caractérisé en ce que** le support comprend un acide polyglycolique (PGA), un acide polylactique (PLA), un polyhydroxyalcanoate (PHA), un poly-4-hydroxybutyrate (P4HB) ou un mélange de deux ou plusieurs de ces polymères.

4. Procédé selon l'une des revendications 1 à 3, **caractérisé en ce que** le support biologiquement dégradable est préalablement fabriqué en forme de valvule cardiaque.

5. Procédé selon l'une des revendications 1 à 3, **caractérisé en ce que** le support biologiquement dégradable est préalablement fabriqué en forme d'un vaisseau sanguin.

6. Procédé selon l'une des revendications 1 à 4, **caractérisé en ce que** le support a une densité de polymère de 40 à 120 mg/cm³, de préférence de 50 à 80 mg/cm³.

7. Procédé selon l'une des revendications 1 à 6, **caractérisé en ce que** le support est un polymère poreux dont la taille des pores est de 80 à 240 µm.

8. Procédé selon l'une des revendications 1 à 7, **caractérisé en ce que** les fibres du support ont un diamètre de 6 à 20 µm, de préférence de 10 à 18 µm.

9. Procédé selon l'une des revendications 1 ou 2, **caractérisé en ce que** le support est une trame de tissu conjonctif d'une valvule cardiaque d'un animal.

10. Procédé selon l'une des revendications 1 ou 2, **caractérisé en ce que** le support est une trame de tissu conjonctif d'un vaisseau d'un animal.

11. Procédé selon l'une des revendications 1 à 10, **caractérisé en ce que** la phase de colonisation est répétée de 3 à 14 fois, de préférence de 5 à 10 fois avec des fibroblastes et/ou des myofibroblastes.

12. Procédé selon l'une des revendications 1 à 11, **caractérisé en ce qu'**on utilise env. 10⁵ à 5 x 10⁸, de préférence 4-5 x 10⁶ fibroblastes et/ou myofibroblastes par centimètre carré de support/matrice et par phase de colonisation.

13. Procédé selon l'une des revendications 1 à 12, **caractérisé en ce qu'**on répète la phase de colonisation de 3 à 14 fois, de préférence de 5 à 10 fois, avec des cellules endothéliales.

14. Procédé selon l'une des revendications 1 à 11, **caractérisé en ce qu'**on utilise env. 10⁵ à 5 x 10⁸, de préférence 4-5 x 10⁶ cellules endothéliales par centimètre carré de support/matrice et par phase de colonisation.

15. Procédé selon l'une des revendications 1 à 14, **caractérisé en ce que** les fibroblastes et/ou les myofibroblastes et/ou les cellules endothéliales sont des cellules humaines.

16. Procédé selon l'une des revendications 1 à 15, **caractérisé en ce que** les fibroblastes et/ou les myofibroblastes et/ou les cellules endothéliales sont des cellules autologues.

17. Procédé selon l'une des revendications 1 à 16,
**caractérisé en ce qu'**on règle, dans la chambre à débit pulsatile, des débits de 50 ml/min à 5000 ml/min, de préférence de 50 à 2000 ml/min.

18. Procédé selon l'une des revendications 1 à 17, **caractérisé en ce que** le débit est augmenté au cours d'une période qui dure de 1 semaine à 12 semaines.

19. Procédé selon l'une des revendications 1 à 18, **caractérisé en ce que** le débit initial est de 50 à 100 ml/min.

20. Procédé selon l'une des revendications 1 à 19, **caractérisé en ce que** la fréquence de pulsation initiale est de 5 à 10 pulsations/min.

21. Procédé selon l'une des revendications 1 à 19, **caractérisé en ce que** le débit est augmenté jusqu'à max. 5000 ml/min pour les prothèses de vaisseau et les prothèses de valvules cardiaques.

22. Procédé selon l'une des revendications 1 à 20, **caractérisé en ce que** la fréquence de pulsations est augmentée jusqu'à max. 180 pulsations/min pour les prothèses de vaisseau et les prothèses de valvules cardiaques.

23. Procédé selon l'une des revendications 1 à 17, **caractérisé en ce qu'**on règle, dans la chambre à débit pulsatile, des pressions systémiques de 10 à 240 mm d'Hg.

24. Valvule cardiaque homologue, pouvant être produite par le procédé selon l'une des revendications 1 à 23.

25. Valvule cardiaque homologue selon la revendication 24, destinée à être implantée chez un patient humain, qui comporte un noyau intérieur de tissu conjonctif entouré d'une couche de cellules endothéliales, et pour laquelle, dans le noyau intérieur en tissu conjonctif, la densité de collagène donnée est de 43 à 55 %.

26. Valvule cardiaque homologue selon la revendication 24, **caractérisée en ce qu'**elle résiste aux conditions de ciculation régnant dans le coeur humain.

27. Vaisseau homologue, pouvant être mis en oeuvre par le procédé selon l'une des revendications 1 à 23.
